# EUROPEAN PATENT SPECIFICATION

(11) **EP 1 732 920 B1**
(45) Date of publication and mention of the grant of the patent: **09.03.2011**
(21) Application number: 04806196.4
(22) Date of filing: 24.12.2004
(51) Int. Cl.: C07D 409/12, C07D 453/02, C07D 333/38, C07D 409/14, C07D 413/12, C07D 413/14, C07D 417/14, A61K 31/41, A61K 31/40, A61K 31/435, A61K 31/495, A61K 31/55, A61P 35/00

(54) **THIOPHENE DERIVATIVES AS CHK 1 INHIBITORS**
THIOPHENDERIVATE ALS CHK-1-INHIBITOREN
DERIVES DU THIOPHENE EN TANT QU'INHIBITEURS CHK1

(30) Priority: 05.01.2004 US 534310 P; 15.03.2004 US 553305 P
(43) Date of publication of application: 20.12.2006
(60) Divisional application: 10184816.6
(73) Proprietor: AstraZeneca AB, 151 85 Södertälje (SE)
(72) Inventor: ASHWELL, Susan, AstraZeneca R & D Boston, Waltham, Massachusetts 02451 (US); GERO, Thomas, AstraZeneca R & D Boston, Waltham, Massachusetts 02451 (US); IOANNIDIS, Stephanos, AstraZeneca R & D Boston, Waltham, Massachusetts 02451 (US); JANETKA, James, AstraZeneca R & D Boston, Waltham, Massachusetts 02451 (US); LYNE, Paul, AstraZeneca R & D Boston, Waltham, Massachusetts 02451 (US); SU, Mei, AstraZeneca R & D Boston, Waltham, Massachusetts 02451 (US); TOADER, Dorin, AstraZeneca R & D Boston, Waltham, Massachusetts 02451 (US); YU, Dingwei, AstraZeneca R & D Boston, Waltham, Massachusetts 02451 (US); YU, Yan, AstraZeneca R & D Boston, Waltham, Massachusetts 02451 (US)
(86) International application number: PCT/GB2004/005400
(87) International publication number: WO 2005/066163

(56) References cited:
- WO-A-03/028731
- WO-A-03/029241
- US-B1- 6 187 799
- ROBBA M ET AL: "Synthèse d'intermédiaires de la thiazolo [4,5-d] pyridazine. II. Amides et hydrazides d'acides thiazole-carboxyliques" BULLETIN DE LA SOCIETE CHIMIQUE DE FRANCE, SOCIETE FRANCAISE DE CHIMIE. PARIS, FR, no. 6, 1969, pages 2152-2157, XP002234183 ISSN: 0037-8968
- CHILDRESS ET AL.: "Thiazolopyrimidines" J. AM. CHEM. SOC., vol. 73, no. 26, 1951, pages 3862-3864, XP002330773
- CHAUVIN ET AL.: "Synthesis of heterocyclic compounds from cyano- and dicyanoselephenes" COMPTES RENDUS DES SEANCES DE DES SCIENCES, SERIE C: SCIENCES CHIMIQUES, vol. 274, no. 14, 1972, pages 1347-1349, XP009048300

## Description

### Field of the invention

The present invention relates to a novel substituted heterocycle, its pharmaceutical compositions and their use in the preparation of a medicament for the treatment and prevention of cancers.

### Background of the invention

Chemotherapy and radiation exposure are currently the major options for the treatment of cancer, but the utility of both these approaches is severely limited by drastic adverse effects on normal tissue, and the frequent development of tumor cell resistance. It is therefore highly desirable to improve the efficacy of such treatments in a way that does not increase the toxicity associated with them. One way to achieve this is by the use of specific sensitizing agents such as those described herein.

An individual cell replicates by making an exact copy of its chromosomes, and then segregating these into separate cells. This cycle of DNA replication, chromosome separation and division is regulated by mechanisms within the cell that maintain the order of the steps and ensure that each step is precisely carried out. Key to these processes are the cell cycle checkpoints (Hartwell et al., Science, Nov 3, 1989, 246(4930):629-34) where cells may arrest to ensure DNA repair mechanisms have time to operate prior to continuing through the cycle into mitosis. There are two such checkpoints in the cell cycle - the G1/S checkpoint that is regulated by p53 and the G2/M checkpoint that is monitored by the Ser/Thr kinase checkpoint kinase 1 (CHK1).

As the cell cycle arrest induced by these checkpoints is a crucial mechanism by which cells can overcome the damage resulting from radio- or chemotherapy, their abrogation by novel agents should increase the sensitivity of tumor cells to DNA damaging therapies. Additionally, the tumor specific abrogation of the G1/S checkpoint by p53 mutations in the majority of tumors can be exploited to provide tumor selective agents. One approach to the design of chemosensitizers that abrogate the G2/M checkpoint is to develop inhibitors of the key G2/M regulatory kinase CHK1, and this approach has been shown to work in a number of proof of concept studies. (Koniaras et al., Oncogene, 2001, 20:7453; Luo et al., Neoplasia, 2001, 3:411; Busby et al., Cancer Res., 2000, 60:2108; Jackson et al., Cancer Res., 2000, 60:566).

Smithkline Beecham Corporation describes 2-ureidothiophene compounds in WO03029241 and 3-ureidothiophene compounds in WO03028731 as CHK1 inhibitors. The present invention provides novel CHK1 inhibitors with improved properties.

### Summary of the invention

In accordance with the present invention, the applicants have hereby discovered a novel compound that is a potent inhibitor of the kinase CHK1 and therefore possesses the ability to prevent cell cycle arrest at the G2/M checkpoint in response to DNA damage. This compound is accordingly useful for its anti-proliferative (such as anti-cancer) activity and is therefore useful in methods of treatment of the human or animal body. The invention also relates to pharmaceutical compositions containing said compound and to its use in the manufacture of medicaments for use in the production of an anti-proliferative effect in warm-blooded animals such as man.

The present invention includes pharmaceutically acceptable salts or prodrugs of said compound. Also in accordance with the present invention applicants provide pharmaceutical compositions and a method to use said compound in the treatment of cancer.

Such properties are expected to be of value in the treatment of disease states associated with cell cycle arrest and cell proliferation such as cancers (solid tumors and leukemias), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acute and chronic inflammation, bone diseases and ocular diseases with retinal vessel proliferation.

### Detailed Description of the Invention

Accordingly, the present invention provides a compound of formula (I) or a pharmaceutically acceptable salt thereof:

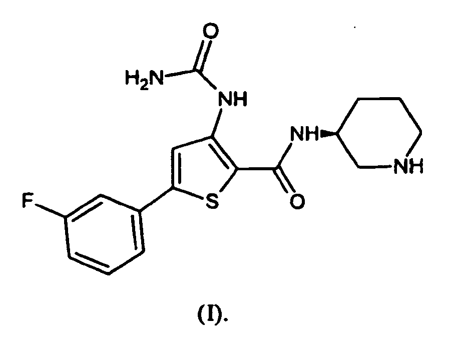

In another embodiment the present invention provides a compound having formula (I) wherein one or more of the atoms is a radioisotope of the same element.

In another embodiment the present invention provides a compound of formula (I) or a pharmaceutically acceptable salt thereof for use in the treatment or prophylaxis of disorders associated with cancer.

In another embodiment the present invention provides a compound of formula (I) or a pharmaceutically acceptable salt thereof for the use in treatment or prophylaxis of neoplastic disease such as cervical cancer, cancer of the head and neck, carcinoma of the breast, ovary, lung(non small cell), pancreas, colon, prostate or other tissues, as well as leukemias and lymphomas, tumors of the central and peripheral nervous system, and other tumor types such as melanoma, fibrosarcoma and osteosarcoma.

In another embodiment the present invention provides a compound of formula (I) or a pharmaceutically acceptable salt thereof for use in the treatment or prophylaxis of proliferative diseases (including autoimmune, inflammatory, neurological, and cardiovascular diseases.

These medical uses are as follows : A method of limiting cell proliferation in a human or animals comprising administering to said human or animal a therapeutically effective amount of a compound of formula (I) or a pharmaceutically acceptable salt thereof.

These medical uses are as follows : A method of inhibiting CHK1 kinase comprising administering to an animal or human in need of said inhibiting a therapeutically effective amount of a compound of formula (I) or a pharmaceutically acceptable salt thereof.

A method of treatment of a human or animal suffering from cancer comprising administering to said human or animal a therapeutically effective amount of a compound of formula (I) or a pharmaceutically acceptable salt thereof.

A method of prophylaxis treatment of cancer comprising administering to a human or animal in need of such treatment a therapeutically effective amount of a compound of formula (I) or a pharmaceutically acceptable salt thereof.

A method of treatment of a human or animal suffering from a neoplastic disease such as cervical cancer, cancer of the head and neck, carcinoma of the breast, ovary, lung (non small cell), pancreas, colon, prostate or other tissues, as well as leukemias and lymphomas, tumors of the central and peripheral nervous system, and other tumor types such as melanomasarcomas including fibrosarcoma and osteosarcoma, malignant brain tumors, comprising administering to said human or animal a therapeutically effective amount of a compound of formula (I) or a pharmaceutically acceptable salt thereof.

A method of treatment of a human or animal suffering from a proliferative disease such as autoimmune, inflammatory, neurological, and cardiovascular diseases comprising administering to said human or animal a therapeutically effective amount of a compound of formula (I) or a pharmaceutically acceptable salt thereof.

A method of treating cancer by administering to a human or animal a compound of formula (I) or a pharmaceutically acceptable salt thereof and an anti-tumor agent.

A method of treating cancer by administering to a human or animal a compound of formula (I) or a pharmaceutically acceptable salt thereof and a DNA damaging agent.

A method for the treatment of infections associated with cancer comprising administering to a human or animal in need of such treatment a therapeutically effective amount of a compound of formula (I) or a pharmaceutically acceptable salt thereof.

A method for the prophylaxis treatment of infections associated with cancer comprising administering to a human or animal in need of such treatment a therapeutically effective amount of a compound of formula (I) or a pharmaceutically acceptable salt thereof.

Another embodiment of the present invention provides a pharmaceutical composition comprising a compound of formula (I) or a pharmaceutically acceptable salt thereof together with at least one pharmaceutically acceptable carrier, diluent or excipent.

In another embodiment the present invention provides the use of a compound of formula (I) or a pharmaceutically acceptable salt thereof in the preparation of a medicament.

In another embodiment the present invention provides the use of a compound of formula (I) or a pharmaceutically acceptable salt thereof in the preparation of a medicament for the treatment or prophylaxis of cancer.

In another embodiment the present invention provides the use of a compound of formula (I) or a pharmaceutically acceptable salt thereof in the preparation of a medicament for the treatment or prophylaxis of neoplastic disease such as carcinoma of the breast, ovary, lung, colon, prostate or other tissues, as well as leukemias and lymphomas including CLL and CML, tumors of the central and peripheral nervous system, and other tumor types such as melanoma, fibrosarcoma and osteosarcoma.

In still another embodiment the present invention provides the use of a compound of formula (I) or a pharmaceutically acceptable salt thereof in the preparation of a medicament for the treatment or prophylaxis of proliferative diseases including autoimmune, inflammatory, neurological, and cardiovascular diseases.

In another embodiment the present invention provides the use of a compound of formula (I) or a pharmaceutically acceptable salt thereof in the preparation of a medicament for use in the inhibition of CHK1 kinase activity.

In another embodiment the present invention provides the use of a compound of formula (I) or a pharmaceutically acceptable salt thereof in the preparation of a medicament for use in limiting cell proliferation.

### Definitions

The definitions set forth in this section are intended to clarify terms used throughout this application. In this section, the definition applies to a compound of formula (I) unless otherwise stated. The term "herein" means the entire application.

As used herein, "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, maleic, tartaric, citric, ascorbic, palmitic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418.

### Combinations

The anti-cancer treatment defined herein may be applied as a sole therapy or may involve, in addition to the compound of the invention, conventional surgery and/or radiotherapy and/or chemotherapy. Such chemotherapy may include one or more of the following categories of antitumour agents:
(i) antiproliferative/antineoplastic drugs and combinations thereof, as used in medical oncology, such as alkylating agents or platinating (for example cis-platin, carboplatin, oxaliplatin, cyclophosphamide, nitrogen mustard, melphalan, chlorambucil, busulphan and nitrosoureas); antimetabolites (for example gemcitabine and fludarabine, as well as antifolates such as fluoropyrimidines like 5-fluorouracil and tegafur, raltitrexed, methotrexate, cytosine arabinoside and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan, irinotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), famesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

### Formulations

Compounds of the present invention may be administered orally, parenteral, buccal, vaginal, rectal, inhalation, insufflation, sublingually, intramuscularly, subcutaneously, topically, intranasally, intraperitoneally, intrathoracially, intravenously, epidurally, intrathecally, intracerebroventricularly and by injection into the joints.

The dosage will depend on the route of administration, the severity of the disease, age and weight of the patient and other factors normally considered by the attending physician, when determining the individual regimen and dosage level as the most appropriate for a particular patient.

An effective amount of a compound of the present invention for use in therapy of infection is an amount sufficient to symptomatically relieve in a warm-blooded animal, particularly a human the symptoms of infection, to slow the progression of infection, or to reduce in patients with symptoms of infection the risk of getting worse.

For preparing pharmaceutical compositions from the compounds of this invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or tablet disintegrating agents; it can also be an encapsulating material.

In powders, the carrier is a finely divided solid, which is in a mixture with the finely divided active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture is then poured into convenient sized molds and allowed to cool and solidify.

Suitable carriers include magnesium carbonate, magnesium stearate, talc, lactose, sugar, pectin, dextrin, starch, tragacanth, methyl cellulose, sodium carboxymethyl cellulose, a low-melting wax, cocoa butter, and the like.

Some of the compounds of the present invention are capable of forming salts with various inorganic and organic acids and bases and such salts are also within the scope of this invention. Examples of such acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Base salts include ammonium salts, alkali metal salts such as sodium, lithium and potassium salts, alkaline earth metal salts such as aluminum, calcium and magnesium salts, salts with organic bases such as dicyclohexylamine salts, N-methyl-D-glucamine, and salts with amino acids such as arginine, lysine, omithine, and so forth. Also, basic nitrogen-containing groups may be quaternized with such agents as: lower alkyl halides, such as methyl, ethyl, propyl, and butyl halides; dialkyl sulfates like dimethyl, diethyl, dibutyl; diamyl sulfates; long chain halides such as decyl, lauryl, myristyl and stearyl halides; aralkyl halides like benzyl bromide and others. Non-toxic physiologically-acceptable salts are preferred, although other salts are also useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

In order to use a compound of the formula (I) or a pharmaceutically acceptable salt thereof for the therapeutic treatment (including prophylactic treatment) of mammals including humans, it is normally formulated in accordance with standard pharmaceutical practice as a pharmaceutical composition.

In addition to the compounds of the present invention, the pharmaceutical composition of this invention may also contain, or be co-administered (simultaneously or sequentially) with, one or more pharmacological agents of value in treating one or more disease conditions referred to herein.

The term composition is intended to include the formulation of the active component or a pharmaceutically acceptable salt with a pharmaceutically acceptable carrier. For example this invention may be formulated by means known in the art into the form of, for example, tablets, capsules, aqueous or oily solutions, suspensions, emulsions, creams, ointments, gels, nasal sprays, suppositories, finely divided powders or aerosols or nebulisers for inhalation, and for parenteral use (including intravenous, intramuscular or infusion) sterile aqueous or oily solutions or suspensions or sterile emulsions.

Liquid form compositions include solutions, suspensions, and emulsions. Sterile water or water-propylene glycol solutions of the active compounds may be mentioned as an example of liquid preparations suitable for parenteral administration. Liquid compositions can also be formulated in solution in aqueous polyethylene glycol solution. Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

The pharmaceutical compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms.

The compound of formula (I) has been shown to inhibit checkpoint kinase activity in vitro. Inhibitors of checkpoint kinase have been shown to allow cells to progress inappropriately to the metaphase of mitosis leading to apoptosis of effected cells, and to therefore have anti-proliferative effects. Therefore it is believed that the compound of formula (I) and its pharmaceutically acceptable salts may be used for the treatment of neoplastic disease as described above. In addition, the compound of formula (I) and its pharmaceutically acceptable salts are also expected to be useful for the treatment of other proliferative diseases. It is expected that the compound of formula (I) would most likely be used in combination with a broad range of DNA damaging agents but could also be used as a single agent.

Checkpoint Kinase 1 Assay: This in vitro assay measures the inhibition of CHK1 kinase by compounds. The kinase domain is expressed in baculovirus and purified by the GST tag. Purified protein and biotinylated peptide substrate (Cdc25C) is then used in a 384 well automated Scintillation Proximity Assay (SPA). Specifically, peptide, enzyme and reaction buffer are mixed and aliquoted into a 384 well plate containing dilution series of compounds and controls. Cold and hot ATP are then added to initiate the reaction. After 2 hours, a SPA bead slurry, CsC12 and EDTA are added to stop the reaction and capture the biotinylated peptide. Plates are then counted on a Topcount. Data is analyzed and IC_{50S} determined for individual compounds.

Abrogation Assay: This cellular assay measures the ability of CHK1 inhibitors to abrogate the DNA-damage induced G2/M checkpoint. Compounds active against the enzyme (< 2 uM) are tested in the cellular assay. Briefly HT29 cells (colon cancer cell line, p53 null) are plated in 96 well plates on day 1. The following day, cells are treated with camptothecin for 2 hours to induce DNA damage. After 2 hours, camptothecin is removed and cells are treated for an additional 18 hours with test compound and nocodazole, a spindle poison that traps in cells in mitosis that abrogate the checkpoint. Cells are then fixed with formaldehyde, stained for the presence of phosphohistone H3, a specific marker for mitosis and labeled with Hoechst dye so that cell number can be measured. Plates are scanned using the Mitotic Index protocol on the Array Scan (Cellomics). As a positive control for abrogation, 4 mM caffeine is used. Compounds are tested in a 12-point dose response in triplicate. Data is analyzed and EC₅₀s determined for individual compounds.

The invention will now be further described with reference to the following illustrative example in which, unless stated otherwise:
(i) temperatures are given in degrees Celsius (°C); operations are carried out at room temperature or ambient temperature, that is, in a range of 18-25°C;
(ii) organic solutions were dried over anhydrous sodium sulfate; evaporation of organic solvent was carried out using a rotary evaporator under reduced pressure 600 - 4000 Pa (4,5 - 30 mm Hg) with a bath temperature of up to 60 °C;
(iii) chromatography means flash chromatography on silica gel; thin layer chromatography (TLC) was carried out on silica gel plates;
(iv) in general, the course of reactions was followed by TLC or liquid chromatography/mass spectroscopy (LC/MS) and reaction times are given for illustration only,
(v) final products have satisfactory proton nuclear magnetic resonance (NMR) spectra and/or mass spectra data;
(vi) yields are given for illustration only and are not necessarily those which can be obtained by diligent process development; preparations were repeated if more material was required;
(vii) when given, NMR data is in the form of delta values for major diagnostic protons, given in part per million (ppm) relative to tetramethylsilane (TMS) as an internal standard, determined at 300 MHz in d₆-DMSO unless otherwise stated;
(viii) chemical symbols have their usual meanings;
(ix) solvent ratio was given in volume:volume (v/v) terms;
(x) Rochelle's Salt is sodium potassium tartarate;
(xi) Hunig's Base is diisopropylethylamine (DIEA);
(xii) Purification of the compounds were carried out using one or more of the following methods:
   a) flash chromatography on regular silica gel;
   b) flash chromatography on silica gel using Isco Combiflash® separation system: RediSep normal phase flash column, flow rate, 30-40 ml/min;
   c) flash chromatography on silica gel using Biotage® separation system;
   c) Gilson semiprep HPLC separation system: YMC pack ODS-AQ column, 100x20mm, S 5µm 12 nm, water (0.1% trifluoroacetic acid) and acetonitrile (0.1% trifluoroacetic acid) as solvents, 20 min run; and
(xvi) the following abbreviations have been used:
   - DMF: dimethylformamide;
   - EtOAc: ethyl acetate;
   - ether: diethyl ether;
   - THF: tetrahydrofuran;
   - MeOH: methanol.

### Example 1

### 5-(3-Fluoro-phenyl)-3-ureido-thiophene-2-carboxylic acid (S)-piperidin-3-ylamide

**methyl 3-({[(trichloroacetyl)amino]carbonyl}amino)thiophene-2-carboxylate.** To a stirred solution of methyl 3-aminothiophene-2-carboxylate (17.0g, 108 mmol) in anhydrous THF (216 mL) was added trichloroacetyl isocyanate (12.9 mL, 108 mmol) slowly over a period of 20 min. After the addition was complete, a precipitate formed and the reaction stirred for an additional 1h at room temperature. The desired product was obtained by filtration to give the title compound (99%) as an off-white solid. The product was used in the next step without any further purification. LC/MS (ES, M+H=345).

**methyl 5-bromo-3-({[(trichloroacetyl)amino]carbonyl}amino)thiophene-2-carboxylate.** To a solution of methyl 3-({[(trichloroacetyl)amino]carbonyl}amino)thiophene-2-carboxylate (10.11 g, 29.38 mmol) in glacial acetic acid (146 mL) at 0°C was added slowly a solution of bromine (3 equiv) in glacial acetic acid (38mL). The resulting cloudy solution was to 70°C for 3 h whereupon the mixture was cooled to rt. The desired product (white solid, 12 g, 97%) was collected by filtration and the filtrate was washed with ether and dried under vacuum. LC/MS (ES, M+H=424).

**methyl 3-[(aminocarbonyl)amino]-5-bromothiophene-2-carboxylate.** A solution of methyl 5-bromo-3-({[(trichloroacetyl)amino)carbonyl}amino)thiophene-2-carboxylate (12g; 28.3 mmol) in dry MeOH (60 mL) was purged with gaseous NH₃ at 0°C until the solution became clear. The mixture was stirred for 20 mins at rt and evaporation gave the desired product (7.8 g, 99%) as white solid. ¹H NMR (300 MHz, DMSO-d6) δ ppm 9.35 (s, 1H), 8.10 (S, 1H), 6.90 br s, 2H), 3.82 (s, 3 H); LC/MS (ES, M+H=280).

***tert*-butyl (3*S*)-3-1[({3-[(aminocarbonyl)amino]-5-bromo-2-thienyl}carbonyl)-amino]piperidine-1-carboxylate.** To a solution of methyl 3-[(aminocarbonyl)amino]-5-bromothiophene-2-carboxylate (1.6 g, 5.7 mmol) in dry THF (30 mL) was added a solution of [Me₃Al/ *tert*-butyl (3*S*)-3-aminopiperidine-1-carboxylate] in THF (18 mL) (which was preformed by the addition of Me₃Al (17.2 mL, 34.4 mmol) to a solution of *tert*-butyl (3*S*)-3-aminopiperidine-1-carboxylate (3.7 g, 17.2 mmol) in THF at -78°C and the resulting yellow solution was stirred at this temperature for 15 mins) and the resulting deep yellow solution was warmed to rt and stirred overnight at this temperature. The reaction mixture was cooled with ice and saturated solution of Rochelle's salt was added to quench the reaction. The mixture was partitioned between EtOAc and H₂O, the aqueous layer was extracted with EtOAc (3x) and the combined organic extracts were washed with H₂O, brine and dried (MSO₄). Evaporation gave a pale orange solid. Purification by Gilson (5%H₂O→95%H₂O-MeCN) gave the desired product (1.0 g, 40%) as off-white solid. ¹H NMR (300 MHz, DMSO-d6; 10.06 (s, 1H), 8.02 (s, 1H), 7.89 (d, 1H), 6.71 (brs, 2H), 4.04 (m, 1H), 3.72 (m, 2H), 2.74 (m, 2H), 1.81 (m, 1H), 1.68 (m, 1H), 1.52 (m, 1H), 1.37 (s, 9H), 1.34 (m, 1H); LC/MS (ES, M+H=448).

### tert-Butyl (3S)-3-({[3-[(aminocarbonyl)amino]-5-(3-fluorophenyl)-2-thienyl]carbonyl}amino)piperidine-1-carboxylate

### (Route A)

*tert*-butyl (3*S*)-3-[({3-[(aminocarbonyl)amino]-5-bromo-2-thienyl}carbonyl)amino]piperidine-1-carboxylate (1,000 mg, 2.24 mmol), 3-fluorobenzeneboronic acid (470.2 mg, 3.36 mmol) and cesium carbonate (2,919.7 mg, 8.96 mmol) were dissolved in a mixture of water (3 mL) and 1,4-dioxane (20 mL). The solution was degassed under low vacuum and nitrogen. Palladium (0) tetrakis triphenylphosphine was added (259 mg, 0.22 mmol) to the solution and the colorless reaction mixture was heated at 75°C with stirring for 1 hour. The aqueous layer was separated and ethyl acetate was added (20 mL) and the resulting solution was dried over MgSO₄ anhydrous.

After solvent evaporation the residual solid was subjected to flash chromatography on silica gel (40 g) with a 2-5% methanol in methylene chloride over 30 min gradient. The main fraction contained traces of triphenylphosphine oxide and was resubjected to flash chromatography under the same conditions to afford a light yellow solid (916 mg, 88 %). MS ES+ 463 (M+1); ¹H NMR (CDCl₃, 300 MHz) δ 10.35 (s, 1H), 8.30 (s, 1H), 7.43-7.28 (m, 3H), 7.05 (m, 1H), 5.91 (brs, 1H), 4.79 (brs, 2H), 4.05 (m, 1H), 3.53-3.55 (m, 2H), 3.36-3.29(m, 2H), 1.89-1.49 (m, 13H).

### (Route B)

**(2*E*,*Z*)-3-chloro-3-(3-fluorophenyl)acrylonitrile.** POCl₃ (13.049 mL, 140 mmol) was added to DMF (21.7 mL, 280 mmol) with stirring and cooling while keeping the temperature below 40°C. After the addition was completed 3-fluoroacetophenone (8.587 mL, 70 mmol) was added slowly. The reaction mixture was heated at 50°C and hydroxylamine chloride was added in portions (19.457 g, 280 mmol). After each addition the temperature was allowed to rise and evolution of gas to cease. When the addition was finished the internal temperature of the reaction mixture reached 140°C. The mixture was allowed to stir and soon solidified. Water/ice was added slowly and the solid that separated was filtered off, was washed with water on the filter (6 × 50 mL) and the solid was taken in ethyl acetate (60 mL), the solution was dried over MgSO₄ anhydrous and solvent was evaporated to afford a brown oil (12 g). The oil was subjected to flash chromatography on silica gel (120 g) with a 15-25 % ethyl acetate in hexanes over 20 min gradient. The early eluting fraction was collected and was evaporated to dryness. The residue was recrystalized from hot petrol ether (150 mL) to afford an off-white solid (3.334 g, 26 %). MS ES+ 182 (M+1); ¹H NMR (CDCl₃, 300 MHz) δ 7.44-7.17 (m, 1H), 7.24-7.17 (m, 1H), 6.04 (s, 1H).

**Methyl 3-amino-5-(3-fluorophenyl)thiophene-2-carboxylate.** Sodium methoxide 25% by weight (3.429 mL, 15 mmol) was dissolved in methanol (6 mL) and methyl thioglycolate (1.341 mL, 15 mmol) was added with stirring. To the brown solution (2*E,Z*)-3-chloro-3-(3-fluorophenyl)acrylonitrile (2.724 g, 15 mmol) was added in small portions and the resulting cream-like mass was heated at 76°C under reflux for 20 min. Ethyl acetate (60 mL) was added to the cold suspension and the organic was washed with water (2 × 50 mL) followed by a dilute solution of bleach (50 mL) and saturated aqueous ammonium chloride (50 mL). The organic was dried over MgSO₄ and solvent evaporated to afford a brown solid. The solid was subjected to flash chromatography on silica gel (120 g) with 20% v/v ethyl acetate in hexanes over 25 min. The first fraction was the desired product that was obtained after evaporation of solvents as an off-white solid (2.282 g, 60.5%). MS ES-250 (M-1); ¹H NMR (CDCl₃, 300 MHz) δ 7.36-7.24 (m, 3H), 7.07-7.06 (m, 1H), 6.76 (s, 1H), 5.48 (brs, 2H), 3.84 (s, 3H).

**Methyl 3-[(aminocarbonyl)amino]-5-(3-fluorophenyl)thiophene-2-carboxylate.** Methyl 3-amino-5-(3-fluorophenyl)thiophene-2-carboxylate (2.517 g, 10 mmol) was dissolved in dry THF and was cooled to -78 °C. Trichloroacetylisocyanate (1.311 mL, 11 mmol) was added with stirring and the reaction was allowed to reach room temperature overnight. Methanol (10 mL) was added slowly and the solvents were evaporated under reduced pressure. The white residue was suspended in methanol (200 mL) and 7M ammonia in methanol was added (3 mL, 21 mmol). After stirring at room temperature for one hour the solvents were evaporated to dryness to afford a white powder. The white powder was suspended in hot methanol (10 mL) and allowed to cool to room temperature. The solid was filtered and dried in the air to afford a cream-colored powder (2.861 g, 97%). MS ES+ 295 (M+1); ¹H NMR (DMSO-d₆, 300 MHz) δ 9.24 (s, 1H), 8.33 (s, 1H), 7.54-7.50 (m, 3H), 7.31-7.24 (m, 1H), 6.86 (brs, 2H), 3.84 (s, 3H).

***tert*-Butyl (3*S*)-3-({[3-[(aminocarbonyl)amino]-5-(3-fluorophenyl)-2-thienyl]-carbonyl}amino)piperidine-1-carboxylate.** Methyl 3-[(aminocarbonyl)amino]-5-(3-fluorophenyl)thiophene-2-carboxylate (1.177 g, 4 mmol) was dissolved in THF (20 mL) and cooled to -78°C. A solution of *tert*-butyl (3*S*)-3-aminopiperidine-1-carboxylate (3.096 mL, 9.6 mmol) in THF (14 mL) was cooled to -78°C and trimethylaluminum 2M in hexanes (5 mL, 10 mmol) was added. An aliquot of the aluminate (20 mL, 10 mmol) was added to the previously prepared solution of ester in THF and the solution was heated at reflux for 4 hours. The cold reaction mixture was washed with 20% aqueous Rochelle's salt. The organic layer was separated and the aqueous layer was extracted with ethyl acetate (2 × 20 mL). The combined organic was dried and solvents were evaporated under reduced pressure. The residue was dissolved in ethyl acetate and was washed with aqueous 1M KHSO₄ (2x 20 mL) and brine (2x 20 mL). After drying over MgSO₄ and solvent evaporation the residue was subjected to flash chromatography on silica gel (120g) with a 50-70% ethyl acetate in hexanes over 45 min gradient. The late eluting fraction was the desired product, *tert*-Butyl (3*S*)-3-({[3-[(aminocarbonyl)amino]-5-(3-fluorophenyl)-2-thienyl]carbonyl}amino)piperidine-1-carboxylate, that was isolated as a glassy solid (724 mg, 39%). MS ES+ 463 (M+1); ¹H NMR (DMSO-d₆, 300 MHz) δ 10.02 (s, 1H), 8.29 (s, 1H), 7.96 (brs, 1H), 7.55-7.42 (m, 3H), 7.24 (t, 1H), 6.67 (brs, 1H), 3.75 (brs, 3H), 2.74-2.72 (m, 2H), 1.83-1.54 (m, 3H), 1.32 (s, 9+2H).

**5-(3-Fluoro-phenyl)-3-ureido-thiophene-2-carboxylic acid (S)-piperidin-3-ylamide.** *tert-Butyl* (3*S*)-3-({[3-[(aminocarbonyl)amino]-5-(3-fluorophenyl)-2-thienyl]carbonyl}amino)piperidine-1-carboxylate (916 mg, 1.98 mmol) was dissolved in methanol (7 mL) and 4M HCl in dioxane (10 mL, 40 mmol) was added while cooling at 0 °C and the solution was stirred for 3hours. The solvent was evaporated under reduced pressure and the residue was taken in methanol (20 mL) and evaporated to dryness. This procedure was repeated 5 times to yield a white powder. The solid was dissolved in water (10 mL) and filtered and solvent was evaporated by lyophilisation over 48 hours. Obtained a light yellow solid (669 mg 84%). MS ES+ 363 (M+1).¹H NMR (DMSO-d₆, 300 MHz) δ 9.95 (s, 1H), 9.23 (brs, 2H), 8.28 (s, 1H), 8.21 (d, 1H), 7.55-7.42 (m, 3H), 7.24 (m, 1H), 4.19 (m, 1H), 3.95 (brs, 6H, water), 3.27-3.14 (m, 2H), 2.90-2.82 (m, 2H), 1.89-1.55 (m, 4H).

## Claims

1. 5-(3-fluoro-phenyl)-3-ureido-thiophene-2-carboxylic acid (S)-piperidin-3-ylamide, or a pharmaceutically acceptable salt thereof.

2. The use of the compound of claim 1 or a pharmaceutically acceptable salt thereof and an anti-tumor agent in the preparation of a medicament for the treatment of cancer.

3. The use of the compound of claim 1 or a pharmaceutically acceptable salt thereof and a DNA damaging agent in the preparation of a medicament for the treatment of cancer.

4. A pharmaceutical composition comprising the compound of claim 1 or a pharmaceutically acceptable salt thereof together with at least one pharmaceutically acceptable carrier, diluent or excipient.

5. The use of the compound of claim 1 or a pharmaceutically acceptable salt thereof in the preparation of a medicament.

6. The use of the compound of claim 1 or a pharmaceutically acceptable salt thereof in the preparation of a medicament for the treatment or prophylaxis of cancer.

7. The use of the compound of claim 1 or a pharmaceutically acceptable salt thereof in the preparation of a medicament for the treatment or prophylaxis of neopiastic disease such as carcinoma of the breast, ovary, lung, colon, prostate or other tissues, as well as leukemias and lymphomas including CLL and CML, tumors of the central and peripheral nervous system, and other tumor types such as melanoma, fibrosarcoma and osteosarcoma.

8. The use of the compound of claim 1 or a pharmaceutically acceptable salt thereof in the preparation of a medicament for the treatment or prophylaxis of proliferative diseases including autoimmune, inflammatory, neurological, and cardiovascular diseases.

9. The use of the compound of claim 1 or a pharmaceutically acceptable salt thereof in the preparation of a medicament for use in the inhibition of CHK1 kinase activity.

10. The use of the compound of claim 1 or a pharmaceutically acceptable salt thereof in the preparation of a medicament for use in limiting cell proliferation.

11. A compound according to claim 1 or a pharmaceutically acceptable salt thereof for use in the treatment or prophylaxis of disorders associated with cancer.

## Patentansprüche

1. 5-(3-Fluorphenyl)-3-ureidothiophen-2-carbonsäure-(S)-piperidin-3-ylamid oder ein pharmazeutisch annehmbares Salz davon.

2. Verwendung der Verbindung nach Anspruch 1 oder eines pharmazeutisch annehmbaren Salzes davon und eines Antitumormittels bei der Herstellung eines Arzneimittels zur Behandlung von Krebs.

3. Verwendung der Verbindung nach Anspruch 1 oder eines pharmazeutisch annehmbaren Salzes davon und eines DNA-schädigenden Mittels bei der Herstellung eines Arzneimittels zur Behandlung von Krebs.

4. Pharmazeutische Zusammensetzung, enthaltend die Verbindung nach Anspruch 1 oder ein pharmazeutisch annehmbares Salz davon zusammen mit mindestens einem pharmazeutisch annehmbaren Träger, Verdünnungsmittel oder Hilfsstoff.

5. Verwendung der Verbindung nach Anspruch 1 oder eines pharmazeutisch annehmbaren Salzes davon bei der Herstellung eines Arzneimittels.

6. Verwendung der Verbindung nach Anspruch 1 oder eines pharmazeutisch annehmbaren Salzes davon bei der Herstellung eines Arzneimittels zur Behandlung oder Prophylaxe von Krebs.

7. Verwendung der Verbindung nach Anspruch 1 oder eines pharmazeutisch annehmbaren Salzes davon bei der Herstellung eines Arzneimittels zur Behandlung oder Prophylaxe von neoplastischen Erkrankungen wie Karzinom der Brust, der Eierstöcke, der Lunge, des Kolons, der Prostata oder anderer Gewebe sowie Leukämien und Lymphomen einschließlich CLL und CML, Tumoren des zentralen und peripheren Nervensystems und anderen Tumortypen wie Melanom, Fibrosarkom und Osteosarkom.

8. Verwendung der Verbindung nach Anspruch 1 oder eines pharmazeutisch annehmbaren Salzes davon bei der Herstellung eines Arzneimittels zur Behandlung oder Prophylaxe von proliferativen Erkrankungen einschließlich Autoimmunerkrankungen, entzündlichen Erkrankungen, neurologischen Erkrankungen und Herz-Kreislauf-Erkrankungen.

9. Verwendung der Verbindung nach Anspruch 1 oder eines pharmazeutisch annehmbaren Salzes davon bei der Herstellung eines Arzneimittels zur Verwendung bei der Inhibierung von CHK-1-Kinase-Aktivität.

10. Verwendung der Verbindung nach Anspruch 1 oder eines pharmazeutisch annehmbaren Salzes davon bei der Herstellung eines Arzneimittels zur Verwendung bei der Einschränkung von Zellproliferation.

11. Verbindung nach Anspruch 1 oder ein pharmazeutisch annehmbares Salz davon zur Verwendung bei der Behandlung oder Prophylaxe von Störungen, die mit Krebs assoziiert sind.

## Revendications

1. (S)-Pipéridin-3-ylamide de l'acide 5-(3-fluoro-phényl)-3-uréido-thiophene-2-carboxylique, ou l'un de ses sels de qualité pharmaceutique.

2. Emploi du composé conforme à la revendication 1 ou de l'un de ses sels de qualité pharmaceutique au titre d'agent antitumoral dans l'élaboration d'un médicament pour le traitement du cancer.

3. Emploi du composé conforme à la revendication 1 ou de l'un de ses sels de qualité pharmaceutique et d'un agent endommageant l'ADN dans l'élaboration d'un médicament pour le traitement du cancer.

4. Composition pharmaceutique comprenant le composé conforme à la revendication 1 ou l'un de ses sels de qualité pharmaceutique en association avec au moins un vecteur, diluant ou excipient de qualité pharmaceutique.

5. Emploi du composé conforme à la revendication 1 ou de l'un de ses sels de qualité pharmaceutique dans l'élaboration d'un médicament.

6. Emploi du composé conforme à la revendication 1 ou de l'un de ses sels de qualité pharmaceutique dans l'élaboration d'un médicament destiné au traitement prophylactique ou thérapeutique du cancer.

7. Emploi du composé conforme à la revendication 1 ou de l'un de ses sels de qualité pharmaceutique dans l'élaboration d'un médicament destiné au traitement prophylactique ou thérapeutique d'une maladie néoplasique telle qu'un carcinome du sein, de l'ovaire, du poumon, du côlon, de la prostate ou d'autres tissus, ainsi que des leucémies et lymphomes, y compris les LLC et les LMC, des tumeurs du système nerveux central et périphérique et d'autres types de tumeurs comme les mélanomes, les fibrosarcomes et les ostéosarcomes.

8. Emploi du composé conforme à la revendication 1 ou de l'un de ses sels de qualité pharmaceutique dans l'élaboration d'un médicament destiné au traitement prophylactique ou thérapeutique de maladies prolifératives, y compris les maladies auto-immunes, inflammatoires, neurologiques et cardio-vasculaires.

9. Emploi du composé conforme à la revendication 1 ou de l'un de ses sels de qualité pharmaceutique dans l'élaboration d'un médicament destiné à l'inhibition de l'activité de la CHK1 kinase.

10. Emploi du composé conforme à la revendication 1 ou de l'un de ses sels de qualité pharmaceutique dans l'élaboration d'un médicament pour emploi dans la limitation de la prolifération cellulaire.

11. Composé conforme à la revendication 1 ou l'un de ses sels de qualité pharmaceutique pour emploi dans le traitement prophylactique ou thérapeutique de troubles associés au cancer.
